# EUROPEAN PATENT SPECIFICATION

(11) **EP 4 023 267 B1**
(45) Date of publication and mention of the grant of the patent: **05.11.2025**
(21) Application number: 20857114.1
(22) Date of filing: 28.07.2020
(51) Int. Cl.: A61L 27/36, A61L 27/12, A61F 2/28

(54) **FORAMINIFERA-DERIVED BONE GRAFT MATERIAL**
KNOCHENTRANSPLANTATMATERIAL AUS FORAMINIFERA
MATÉRIAU DE GREFFE OSSEUSE DÉRIVÉ DE FORAMINIFÈRE

(30) Priority: 30.08.2019 KR 20190107653
(43) Date of publication of application: 06.07.2022
(73) Proprietor: Cellco Inc., Jeonju-si, Jeolabuk-do 55069 (KR)
(72) Inventor: KIM, Beom Su, Iksan-si, Jeollabuk-do 54666 (KR); PARK, Ho, Iksan-si, Jeollabuk-do 54661 (KR)
(74) Representative: Comoglio, Elena
(86) International application number: PCT/KR2020/009933
(87) International publication number: WO 2021/040249

(56) References cited:
- JP-A- 2009 511 420
- KR-A- 20110 088 903
- KR-B1- 102 100 961
- ROY DELLA 1 ET AL: "Hydroxyapatite formed from coral skeletal carbonate by hydrothermal exchange", NATURE, 1 January 1974 (1974-01-01), pages 220 - 222, XP093069268, Retrieved from the Internet <URL:https://www.nature.com/articles/247220a0> [retrieved on 20230801]
- BEN-NISSAN ET AL: "Chapter 3: Marine derived biomaterials for bone regeneration and tissue engineering: learning from nature", 9 July 2019 (2019-07-09), XP009526344, ISSN: 2195-0644, ISBN: 978-981-13-8855-2, Retrieved from the Internet <URL:http://link.springer.com/10.1007/978-981-13-8855-2_3> [retrieved on 20190709]
- BEN-NISSAN, BESIM ET AL.: "Marine derived biomaterials for bone regeneration and tissue engineering: learning from nature", SPRINGER SERIES IN BIOMETERIALS SCIENCE AND ENGINEERING BOOK SERIES, vol. 14, 9 July 2019 (2019-07-09), pages 51 - 78, XP009526344, ISBN: 978-981-13-8855-2, DOI: 10.1007/978-981-13-8855-2_3
- MOHD PU'AD N.A.S., KOSHY P., ABDULLAH H.Z., IDRIS M.I., LEE T.C.: "Syntheses of hydroxyapatite from natural sources", HELIYON, vol. 5, no. 5, 1 May 2019 (2019-05-01), pages e01588, XP055785801, ISSN: 2405-8440, DOI: 10.1016/j.heliyon.2019.e01588
- CHOU JOSHUA, BEN-NISSAN BESIM, GREEN DAVID W., VALENZUELA STELLA M., KOHAN LAWRENCE: "Targeting and Dissolution Characteristics of Bone Forming and Antibacterial Drugs by Harnessing the Structure of Microspherical Shells from Coral Beach Sand", ADVANCE ENGINEERING MATERIALS, WILEY VCH VERLAG, WEINHEIM., DE, vol. 13, no. 1-2, 1 February 2011 (2011-02-01), DE, pages 93 - 99, XP055785806, ISSN: 1438-1656, DOI: 10.1002/adem.201000208

## Description

### TECHNICAL FIELD

The present disclosure relates to a foraminifera-derived bone graft material.

### BACKGROUND ART

In general, when a bone tissue is damaged due to trauma, deformity, or physiological phenomena, guided bone regeneration (GBR), which is a procedure of filling a damaged bone tissue site with a bone graft material and covering it with a barrier membrane to prevent soft tissue intervention and to generate new bones, is performed. The most common GBR methods for restoration of a bone defect include an autograft method in which a portion of an individual's own bone is harvested from somewhere else in the body and grafted onto the body of the same individual, an allograft method in which a bone of someone else is chemically treated and grafted onto the body of an individual, and a xenograft method in which a bone of an animal is chemically treated and grafted onto the human body.

In the case of an autogenous bone graft material used for the autograft method in which a portion of an individual's own bone is harvested and grafted, autogenous cancellous bone (ACB) is generally grafted so that there is almost no immune rejection response and little bone resorption, resulting in excellent osteoconduction and osteoinduction abilities. In the case of an allogenous bone graft material and a xenogeneic bond graft material that are used for the allograft method and the xenograft method, respectively, there is an advantage of eliminating the need for secondary surgery on sites other than a site of bone loss of a graftee, which is concerned as a disadvantage of the autogenous bone graft material. This is because the allograft method uses the bone of an individual other than a graftee and the xenograft method uses the bone of an animal other than a human.

The allogeneic bone which is obtained from a dead body or other living donors is deep-frozen or subjected to freeze-drying, demineralized freeze-drying, and irradiation, so as to remove antigenicity for use. Although the allogeneic bone requires a long period of time for the bone formation and the amount of newly formed bones is small, it is available anytime in a desired amount and does not create an additional surgical site. Types of the allogeneic bone include a demineralized freeze dried bone allografts (DFDBA), a freeze dried boneallografts (FDBA), and an irradiated cancellous bone (ICB).

The xenogeneic bone is a graft material that expects osteoconduction ability by dropping immune responses through various processes after collecting bones from animals such as cattle or pigs. Despite of the advantages of not creating an additional surgical site and being sufficiently available in a desired amount, it takes long for absorption and substitution. Thus, such a mechanism currently tends to be understood in terms of osteoconduction rather than osteoinduction. Types of the xenogeneic bone are Bio-Oss, ABM/P-15, and BioCeraTM.

An alloplastic bone is not a real bone, but an artificially synthesized bone. In this regard, it has poor quality and requires a long period of time for the bone formation, as compared to other bone graft materials, but is inexpensive. For example, non-porous hydroxyapatite (HAp), HAp cement, porous HAp, beta tricalcium phosphate, polymethylmethacrylate (PMMA), hydroxyet-hylmethacrylate (HEMA) polymer, and bioactive glass are being used in clinical practice. Among these examples, HA, PMMA, and HEMA polymer are non-absorbent, whereas tricalcium phosphate and bioactive are absorbent. Examples of the alloplastic bone are tricalcium phosphate, hard tissue polymer, and bioactive glassy ceramics. Here, hydroxyapatite (HAp) has been widely used as a bone substitute in bone grafts and dental devices. HAp is a biocompatible and bioactive material having osteoconduction properties, and a chemical composition of HAp is similar to that of a natural bone tissue. HAp is a kind of calcium phosphate bioceramic, and may be synthesized by using a chemical substance containing a calcium ion and a phosphate ion as raw materials. Several reports on the preparation of HAp ceramics from marine algae via a hydrothermal reaction have been reported.

In Marine-Derived Biomaterials for Tissue Engineering Application (2021) Chapter 3, pages 51-78 bone graft material prepared by a hydrothermal treatment of foraminifera shells is disclosed.

Accordingly, there is a need to develop a material having high cell proliferation and excellent adhesion for use as a bone graft material.

### TECHNICAL PROBLEM

An aspect of the present disclosure provides a bone graft material with a unique structure having remarkable cell proliferation, cell adhesion, and osteoblast differentiation abilities and including a structure capable of supporting newly formed bones.

Another aspect of the present disclosure provides a method of preparing the bone graft material.

### SOLUTION TO PROBLEM

The invention is defined in the appended claims. Various aspects are further disclosed herein. Embodiments falling outside the scope of the claims are not part of the invention. An aspect of the present disclosure provides a bone graft material comprising hydroxyapatite, wherein the hydroxyapatite comprises a plurality of chambers separated by partition walls, and the partition walls comprise a plurality of pores.

The hydroxyapatite is derived from foraminifera (for example, the exoskeleton of foraminifera).

The foraminifera may refer to a protozoan of rhizopod having a shell. Examples of the foraminifera are genus Globigerina, genus Camerina, genus Elphidium, genus Myogipsina, genus Baculogypsina, and the like. In particular, the genus Baculogypsina may be *Baculogypsina sphaerulata, Baculogypsina bonarellii, Baculogypsina gallowayi, Baculogypsina lenticulate, Baculogypsina meneghinii, Baculogypsina saoneki,* or *Baculogypsina sphaerica.*

The term "bone graft material" as used herein may refer to a material that can be used for preservation of bone defects, stimulation of bone formation, joint fusion, and prevention of joint braking and dislocation, and that can be used in bone grafting. Therefore, in the present specification, the bone graft material may be used for treatment of bone defects.

In the present specification, the bone graft material may be, for example, applicable to ethmoid, frontal, nasal, occipital, parietal, temporal, mandible, maxilla, zygomatic, cervical vertebra, thoracic vertebra, lumbar vertebra, sacrum, rib, sternum, clavicle, scapula, humerus, radius, ulna, carpal bones, metacarpal bones, phalanges, ilium, ischium, pubis, femur, tibia, fibula, patella, calcaneus, tarsal, and metatarsal bones.

In addition, in the present specification, the bone graft material may be used in dentistry (dental implants), plastic surgery, or orthopedic surgery.

The term "osteogenesis ability" as used herein refers to a function of inducing or promoting bone matrix formation and osteoanagensis by osteoblasts, and includes all the cartilaginous osteogenesis, connective tissue osteogenesis, and transformed osteogenesis.

The term "osteoconduction ability" as used herein refers to a function of attracting osteoblasts to induce or promote bone matrix formation by the osteoblasts.

The term "osteoinduction ability" as used herein refers to a function of inducing regeneration of a desired bone tissue by accessing only cells and materials useful for bone tissue regeneration to bone defects.

The terms "administering", "introducing", "grafting" are used interchangeably, and may refer to arrangement of a composition according to an embodiment in a subject by a method or pathway that results in at least partial localization of the composition to a desired site. Cells or at least some of cell components of the composition according to an embodiment may be administered by any suitable pathway that delivers to a desired location in a living subject.

In an embodiment, the bone graft material may comprise a plurality of hydroxyapatite particles. Here, the particle size may be in a range of 50 µm to 4,000 µm, 50 µm to 3,000 µm, 50 µm to 2,000 µm, 80 µm to 2,000 µm, 100 µm to 4,000 µm, 100 µm to 2,000 µm, 100 µm to 1,500 µm, 100 µm to 1,200 µm, 100 µm to 1,000 µm, 100 µm to 700 µm, or 120 µm to 600 µm.

In an embodiment, the chamber may have a diameter in a range of 5 µm to 200 µm, 5 µm to 180 µm, 5 µm to 150 µm, 5 µm to 120 µm, 10 µm to 100 µm, 10 µm to 80 µm, 10 µm to 60 µm, or 15 µm to 60 µm. The chamber may have a cross-sectional area in an elliptical shape, and the diameter may refer to a short diameter and/or a long diameter of the ellipse.

In an embodiment, the pore may have a diameter in a range of 0.05 µm to 5 µm, 0.05 µm to 4.5 µm, 0.05 µm to 4 µm, 0.08 µm to 3 µm, 0.08 µm to 2 µm, 0.1 µm to 3 µm, 0.1 µm to 2 µm, or 0.2 µm to 2 µm.

In another embodiment, the partition wall may have a thickness in a range of 1 µm to 50 µm, 1 µm to 45 µm, 1 µm to 40 µm, 2 µm to 40 µm, 4 µm to 40 µm, 4 µm to 30 µm, 5 µm to 30 µm, 5 µm to 20 µm, or 5 µm to 15 µm.

The hydroxyapatite has per 1 cm² of the surface thereof, 40,000 uniform chambers to 60,000 uniform chambers.

In another embodiment, the hydroxyapatite particle may comprise 0.5 weight%(atomic%) to 10 weight%(atomic%) of magnesium ions, 0.2 weight%(atomic%) to 10 weight%(atomic%) of silicon ions, or 0.1 weight%(atomic%) to 5 weight%(atomic%) of strontium ions. The term "weight%" as used herein refers to a weight percent and is typically expressed as a weight percentage of the total weight.

The hydroxyapatite is prepared by performing a hydrothermal reaction on the exoskeleton of foraminifera.

The term "hydrothermal synthesis" as used herein is also referred to as a hydrothermal reaction, and refers to a synthesis reaction of a substance in the presence of water at high temperatures or water under high temperatures and high pressures.

The hydroxyapatite in the bone graft material is prepared by performing a hydrothermal reaction on a pretreated exoskeleton of foraminifera at a temperature of 100 °C to 300 °C, for 12 hours to 36 hours.

In another embodiment, hydroxyapatite in the bone graft material disclosed herein may be prepared by treating a pretreated exoskeleton of the foraminifera with microwaves.

In an embodiment, the bone graft material may or may not include tricalcium phosphate (TCP).

The term "not including" refers to "substantially not including", and the term "substantially" as used herein refers to inclusion of a material in an amount that does not affect the activity of the bone graft material.

In another embodiment, the bone graft material may have cell proliferation ability, cell adhesion ability, or osteoblast differentiation ability.

In an embodiment, the bone graft material may further comprise cells, for example, somatic cells or stem cells.

The term "stem cells" as used herein may refer to undifferentiated cells having ability to differentiate into other cells. The stem cells may be embryonic stem cells, adult stem cells, induced pluripotent stem cells, or mesenchymal stem cells. The mesenchymal stem cells may be isolated from various tissues, various races, or people of different ages. For example, the mesenchymal stem cells may be adipose tissue-derived, placenta-derived, cord blood-derived, muscle tissue-derived, corneal tissue-derived, or bone marrow tissue-derived mesenchymal stem cells. In addition, for example, the mesenchymal stem cells may be adipose stem cells, bone marrow stem cells, cord blood stem cells, neural stem cells, placenta stem cells, or cord blood stem cells.

The bone graft material disclosed herein may further comprise a binding agent. The term "binding agent" as used herein may refer to a material that physically fixes, binds, or coagulates hydroxyapatite to prevent the bone graft material from immediately leaving a bone defect when applied to the bone defect. The binding agent may comprise a biocompatible material known in the art, and examples thereof are: dental resin cements; glass ionomer cements; collagen-based glues; phosphate-based cements such as zinc phosphate, magnesium phosphate, and the like; zinc carboxylate; and protein-based binders such as fibrin glues, mussel-derived adhesive proteins, and the like.

The bone graft material disclosed herein may further comprise an additive. The additive may be a medically acceptable additional component that is added to prevent infection of a bone defect or to further improve osteogenesis, osteoconduction, or osteoinduction ability of the bone graft material. Examples of the additive are: drugs such as antiviral agents, antibacterial agents, antibiotics, anticancer agents, and angiogenic drugs; polysaccharide aqueous solutions; amino acids; peptides; vitamins; cofactors for protein synthesis; growth hormones hormones such as somatotropin; endocrine tissue fragments; enzymes such as collagenase, peptidases, and oxidases; living cells such as cartilage fragments, chondrocytes, and bone marrow cells; immunosuppressants; fatty acid esters; and nucleic acids, but are not limited thereto. A person skilled in the art may select one or more suitable additives depending on a bone defect site and conditions of a subject.

The bone graft material disclosed herein may be formulated in the form of a powder, a suspension, an emulsion, an ointment, or an injection according to a conventional method, and then applied to a bone defect site or a surrounding area thereof. The term "dosage" as used herein refers to an amount sufficient to induce or promote formation or regeneration of bones when applied to a target site in need thereof. The dosage of the bone graft disclosed herein may vary depending on weight, age, gender, and health status of a patient, and degree of bone defects, and may be appropriately selected by a person skilled in the art.

In addition, another aspect of the present disclosure provides a composition for preparing the bone graft material.

The composition for preparing the bone graft material disclosed herein may further comprise one or more selected from the group consisting of water, saline, sterile water, Ringer's solution, buffered saline, cyclodextrin, dextrose solution, maltodextrin solution, glycerol, ethanol, liposome, lactose, dextrose, sucrose, sorbitol , mannitol, xylitol, erythritol, maltitol, starch, acacia gum, alginate, gelatin, calcium phosphate, calcium silicate, cellulose, methylcellulose, polyvinylpyrrolidone, methylhydroxybenzoate, propylhydroxybenzoate, talc, magnesium stearate, mineral oil, calcium carbonate, dextrin, propylene glycol, and liquid paraffin.

Another aspect of the present disclosure provides a method of preparing the bone graft material.

The method comprises pre-treating foraminifera; preparing hydroxyapatite by performing a hydrothermal reaction on the pretreated exoskeleton of foraminifera at a temperature in a range of 100 °C to 300 °C for 12 hours to 36 hours and optionally preparing hydroxyapatite by treating the pretreated exoskeleton of foraminifera with microwaves; and sintering the hydroxyapatite.

The pre-treating of foraminifera may comprise: washing the foraminifera; and/or adding the washed foraminifera to an aqueous solution containing a compound capable of providing phosphoric acid.

The compound capable of providing phosphoric acid may be (NH₄)H₂PO₄, H₃PO₄, Na₃PO₄, or Na₂HPO₄.

In addition, the preparing of hydroxyapatite by treating with microwaves may comprise treating with a microwave in a wavelength range of 300 MHz to 300 GHz in an amount of 100 W to 1,500 W for 0.5 minute to 48 hours.

In addition, the sintering comprises the steps of raising the temperature to 400 °C to 800 °C at a rate of 1 °C/min to 20 °C/min, maintaining at the same temperature for 1 hour to 8 hours, and raising the temperature again to 600 °C to 1,500 ° at a rate of heating again with 1 °C/min to 20 **°**C/min for 1 hour to 8 hour. The cooling is performed until the temperature is lowered to room temperature.

### ADVANTAGEOUS EFFECTS OF DISCLOSURE

According to a foraminifera-derived bone graft material as disclosed herein, the foraminifera-derived bone graft material has remarkable cell proliferation, cell adhesion, and osteoblast differentiation abilities, and includes a structure capable of supporting newly formed bones, so that it can be effectively used as a bone graft material.

### BRIEF DESCRIPTION OF THE DRAWINGS

FIG. 1 shows scanning electron microscopy images of a structure of hydroxyapatite particles according to an embodiment; A: 1,000 X, 10,000 X, B: 100 X, 1,000 X, 3,000 X.
FIG. 2 shows scanning electron microscopy images showing the measured size of the chambers and pores of hydroxyapatite particles according to an embodiment.
FIG. 3 shows comparison results of the XRD analysis of hydroxyapatite particles according to an embodiment with a control group HAp.
FIG. 4 is an image showing the cytotoxicity results measured from hydroxyapatite according to an embodiment.
FIG. 5 is a graph showing the cell proliferation results measured from hydroxyapatite according to an embodiment.
FIG. 6 is a scanning electron microscopy image showing the cell adhesion and infiltration results measured from hydroxyapatite according to an embodiment.
FIG. 7 is a graph showing the ALP activity analysis results measured from hydroxyapatite according to an embodiment.
FIG. 8 is a graph showing the expression levels of osteoblast marker genes in hydroxyapatite according to an embodiment.
FIG. 9 shows a figure (A) showing the micro-CT results at the 8^{th} week of in vivo graft of hydroxyapatite according to an embodiment and a graph (B) showing quantified data thereof.
FIG. 10 shows the H&E staining results showing in vivo osteoanagenesis effects of hydroxyapatite according to an embodiment.
FIG. 11 shows the Goldner's Masson trichrome staining results showing in vivo osteoanagenesis effects of hydroxyapatite according to an embodiment.

### MODE OF DISCLOSURE

Hereinafter, the present disclosure will be described in detail with reference to Examples below. However, these Examples are provided for illustrative purposes only, and the scope of the present disclosure is not limited thereto.

### Reference Example 1. Cell culture

The biocompatibility of foraminifera-derived hydroxyapatite (HAp) (also, referred to as foraminifera-HAp) was evaluated by using human mesenchymal stem cells (hMSCs). In detail, cells were cultured under conditions of 5 % CO₂ and 37 °C in α-MEM (Gibco-BRL, MD, Gaithersburg, MD) supplemented with 10 % fetal bovine serum (FBS; Gibco-BRL), wherein the cells subcultured from passage 4 to passage 6 were used for *in vitro* experiments.

To induce osteoblasts differentiation, the cells were cultured in a medium supplemented with an osteogenic stimulator (0.1 mM of dexamethasone, 0.1 M of β-glycerophosphate, and 50 µg/mL of ascorbic acid). All compounds used in the medium supplemented with the osteogenic stimulator were cell culture-grade reagents (Sigma Aldrich, St. Louis, MO, USA). A normal growth medium and the medium supplemented with the osteogenic stimulator were replaced with fresh media every 2 days during the experiments.

Before the cell culture experiments, HAp particles were first sterilized with 70 % alcohol and washed with a phosphate buffer solution (PBS) three times for 10 minutes. After the sterilization, 20 mg of the sterilized pure HAp particles and foraminifera-derived HAp particles were added to a 48-well culture plate. The cells were seeded (at a concentration of 5 x 10⁴ cells/well) and cultured for 2 hours so that the initial cells were attached to each HAp particle. The HAp particles to which the cells were attached were transferred to a new culture plate, and then cultured for *in vitro* experiments.

### Reference Example 2. Cell proliferation

The cell proliferation was evaluated according to mitochondrial activity-based analysis using CellTiter96^{®} Aqueous One solution (MTS assay, Invitrogen, Carlsbad, CA, USA). As described above, the hMSCs were seeded and cultured for 1 day, 3 days, 6 days, 9 days, and 12 days. At a predetermined point, 50 µL of a CellTiter96^{®} reagent solution was mixed with 250 µL of the normal medium, and the mixture was added to each well. After 4 hours of the cell culture, the supernatant was collected to measure absorbance at 490 nm by using an ELISA plate reader (SpectraMAX M3; Molecular Devices, Sunnyvale, CA).

### Reference Example 3. Cell viability and cytotoxicity

The cell viability and cytotoxicity were evaluated according to fluorescence staining by using Live/Dead^{®} and a viability/cytotoxicity kit (Invitrogen, Carlsbad, CA, USA). According to the preparation protocol, the cultured HAp particles were washed with a PBS for 30 minutes. Subsequently, the cells were stained by using calcein acetoxymethyl ester (Calcein AM) and ethidium homodimer-1(EthD-1) of the kit, and then observed with an inverted fluorescence microscope (DM IL LED Fluo; Leica Microsystems, Wetzlar, Germany).

### Reference Example 4. In vitro evaluation of cell adhesion and cell infiltration

To observe the cell adhesion on the surface and the cell infiltration to each HAp particle, the cells were cultured for 5 days. A specimen was washed with a PBS, fixed in 2.5 % glutaraldehyde solution at 4 °C for 2 hours, and post-fixed with 0.1 % osmium tetroxide solution. Next, the specimen was dehydrated via graded ethanol series (30 %, 50 %, 75 %, 85 %, 95 %, and 100 %, each for 10 minutes). Then, the resulting specimen was sputter-coated with gold, and then observed with an electronic microscope (EM; EM-30). To observe the cell infiltration, the HAp particles were sliced with a sharp lancet to expose cross sections of the surface, followed by SEM analysis.

### Reference Example 5. Measurement of alkaline phosphatase (ALP) activity

The osteoblast differentiation was evaluated by ALP activity analysis. The ALP activity analysis was performed by using p-nitrophenylphosphate (p-NPP) as a substrate. In detail, the hMSCs were seeded on the particles, and then cultured in the medium supplemented with the osteogenic stimulator. After 5 days of the cell culture, adhesive cells were lysed by sonication in 1 % Triton X-100/PBS solution under an icebox condition. To remove the particles and residues, the sample was centrifuged at 4 °C at a speed of 12,000 rpm. The supernatant was used for the ALP activity analysis and the protein concentration analysis. In the present study, the ALP activity was normalized to total protein contents.

### Reference Example 6. Real-time polymerase chain reaction

To measure the osteoblast differentiation of the hMSCs cultured on the HAp particles, several osteogenesis marker genes, such as ALP, collagen type Iα1 (Col1αl), osteocalcin (OCN), and bone sialoprotein (BSP), were measured by quantitative real-time polymerase chain reaction (RPCR). After the cells were cultured for 7 days in the medium supplemented with the osteogenic stimulator, total mRNAs were isolated from the cells, and cDNAs were transcribed with a reverse transcriptase (Invitrogen) and oligo (dT) primers. Then, the cDNAs were amplified with TaqMan Universal PCR Master mix(Applied Biosystem) and primers and TaqMan probe sets for ALP (Hs01029144_m1), Col1αl (Hs00164004_m1), OCN (Hs01587814_g1), BSP (Hs00173720_m1), and 18S (Hsscience). All TaqMan PCRs were performed by using a StepOne Plus RPCR system (Applied Biosystems, Foster City, CA, USA), and 18S rRNA gene was co-amplified as an internal standard.

### Reference Example 7. Animal model

4 adult male New Zealand white rabbits (age > 3 months or more, 2.5 kg to 3.0 kg) were anesthetized with intramuscular doses of ketamine (35 mg/kg; Yuhan Corporation, Seoul) and xylazine (5 mg/kg; Bayer Korea, Seoul). Then, 2 % lidocaine solution was used for local anesthesia. After tissue excision, three isolated circular cranial defects were made by using trephine with an outer diameter of 6 mm. One defect was used as a control group, another defect was filled with pure HAp particles, and the other defect was filled with foraminifera-derived HAp particles. The healing process was observed 8 weeks after the graft, and the defects and the surrounding bones thereof were incised from host bones. Then, before performing further analysis, the specimen was removed and placed in a fixing solution (phosphate buffered 4 % paraformaldehyde solution, pH 7.2) at 4 °C for 7 days.

### Reference Example 8. Bone analysis by micro-CT

The newly formed bones were evaluated pathologically by micro-CT (Sky-Scan 1172TM, Skyscan, Kontich, Belgium). At the 8^{th} week after the graft, the sample was analyzed by using an aluminum filter (0.5 mm) with X-ray set at a voltage of 60 kV and a current of 167 µA. Based on the micro-CT results, the qualitative images of the defects were observed with 3D reconstruction images based on a 3D software (CTVol, Skyscan), and the percent bone volume (BV) was calculated as follows by using an image analysis software (CT-analyzer TM, Skyscan).BV (%) = (volume of new bones)-(volume of remaining graft material)/total defect volume

### Reference Example 9. Histological analysis

The histological analysis was performed at the 8^{th} week after the graft. The fixed specimen of a rat cranium was treated with 8 % formic acid/8 % HCl to remove calcium deposit, and then dehydrated with graded alcohol series (70 % to 100 %). Finally, the specimen was placed in paraffin. Each specimen was cut into 5 µm sections by using a rotary microtone (HM 325^{™}; Microm, Walldorf, Germany). Five sections from the center of each sample were stained with hematoxylin-eosin (HE) and Goldner's masson trichromand (MT). Then, the samples were randomly selected, and the formation of new bones was observed under a microscope (DMR, Leica, Nussloch, Germany).

### Reference Example 10. Statistical analysis

The numerical values are expressed as mean ± standard deviation (SD), and the statistical analysis was performed by one-way analysis of variance (ANOVA). Then, the Dunnett's post-hoc test was performed by using GraphPad Prism version 5.3 (GraphPad Software, SanDiego, CA, USA), wherein P <0.05 was considered statistically significant.

### Examples. Preparation and analysis of hydroxyapatite particles

### 1. Preparation of hydroxyapatite (HAp) particles by using foraminifera

The hydroxyapatite by using foraminifera were prepared as follows.

First, foraminifer (Baculogypsina sphaerulata, Okinawa, Japan) was purchased in the market. To remove residual contaminants and organic ingredients, the sample was boiled with 4 % sodium perchlorate (NaClO₄) and washed with distilled water. In detail, the sample was added to an ammonium phosphate monobasic ((NH₄)H₂PO₄) aqueous solution, wherein a molar ratio of Ca:P was 10:6.

Subsequently, the sample was added to a Teflon-lined stainless autoclave, and heated at 200 °C for 24 hours. The transformed sample was washed with boiling water, and dried at 60 °C. Then, HAp particles were chopped with a lancet, and HAp particles having a diameter in a range of 200 µm to 500 µm were separated by filtration through a stainless mesh. Afterwards, the crystallization was performed on the separated particles by a sintering process. The sintering was performed in an electric furnace (Muffle Furnace, SH-FU-4MH), wherein, after the temperature was raised to 600 °C at a heating rate of 5 °C /min, the same temperature was maintained for 2 hours. Afterwards, the temperature was raised again to 800 °C at a heating rate of 5 °C /min, and the same temperature was maintained for 4 hours. Then, the temperature was lowered to room temperature, thereby completing the sintering progress.

Here, as a control group, HAp particles (pure HAp particles having a particle size in a range of 200 µm to 500 µm) that were stoichiometrically synthesized were obtained from Dio Implant Inc. (Busan, Korea) and used.

### 2. Chemical and morphological characteristics

To analyze compositions of the foraminifera-derived HAp of Example 1, the X-ray diffraction analysis (XRD; D8, Bruker AXS, Karlsruhe, Germany) using CuKα radiation was performed under conditions of a scanning rate of 0.02°/minute, voltage of 50 kV, and currency of 30 mÂ in a range of 10° to 80°. The ionic compositions of the sample was evaluated by X-ray fluorescence (XRF, Bruker) spectroscopy. The microstructure and surface morphology of the foraminifer-derived HAp particles were observed under vacuum by using a scanning electron microscope (SEM; EM-30, COXEM, Daejeon, Korea).

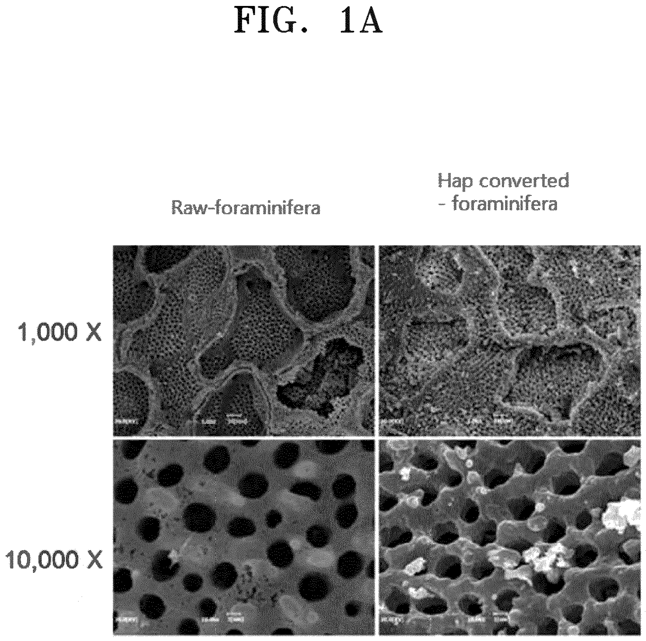

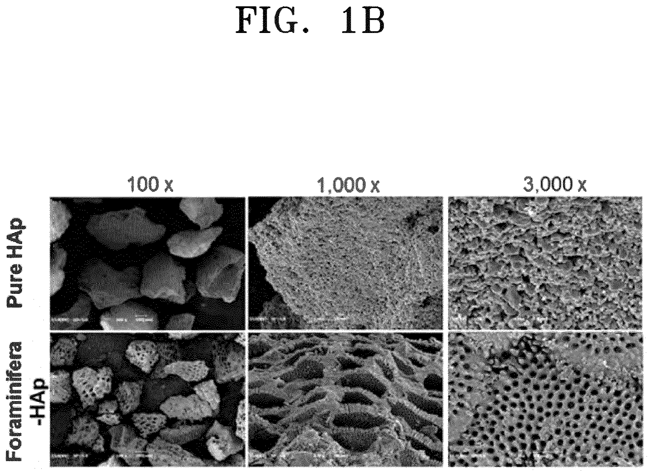

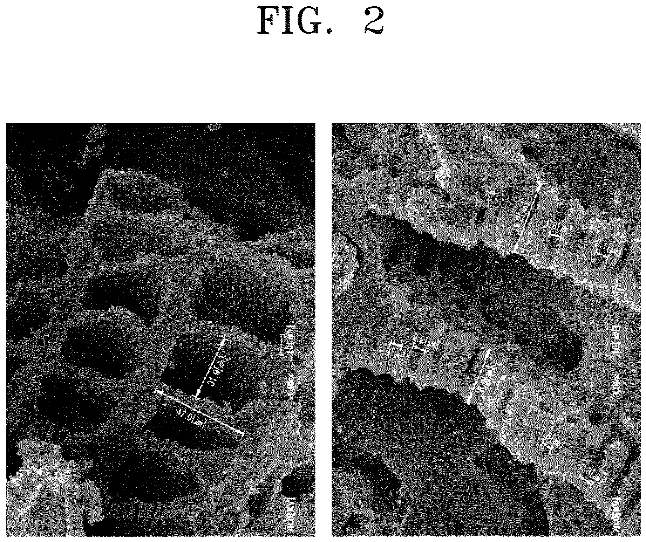

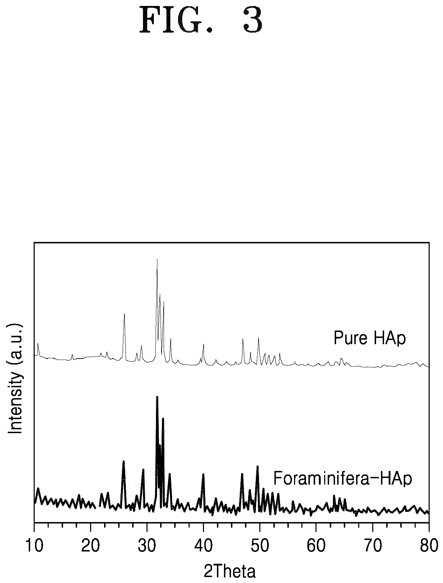

The SEM images obtained by observation are shown in FIGS. 1 and 2, and the XRD analysis results are shown in FIG. 3. The ionic compositions of the sample are shown in Table 1.

**Table 1**

| Element | Amount (%) |
|---|---|
| Mg | 2.98 % |
| Si | 1.53 % to 2.5 % |
| Sr | 0.52 % to 1.11 % |

FIG. 1 shows the SEM images of the structure of hydroxyapatite particles according to an embodiment. FIG. 2 shows the SEM images showing the measured size of the chambers and pores of hydroxyapatite particles according to an embodiment.

FIG. 3 shows comparison results of the XRD analysis of hydroxyapatite particles according to an embodiment with a control group HAp.

As shown in FIGS. 1 and 2, the SEM images of the pure HAp particles show densely packed HAp blocks and surface morphology with mostly non-micron-sized porosity. Meanwhile, hydroxyapatite according to an embodiment had a plurality of chambers separated by partition walls having pores, and thus had a macro-sized pore structure divided by the plurality of chambers that were interconnected inside. In addition, it was confirmed that the pores were uniformly distributed on the surface of the particles. In addition, no significant morphological change was found, and such morphological characteristics were maintained during the transformation into HAp.

In addition, as shown in FIG. 2, the HAp had about 53,300 uniform chambers per 1 cm², and the size of the chamber was about 50 µm * 25 µm. In addition, the size of the pore of the partition wall was ~ 2 µm.

As shown in FIG. 3, it was confirmed that the peak patterns after the hydrothermal reaction were consistent with hydroxyapatite according to an embodiment and commercially available pure HAp. These results indicate that foraminifera was successfully transformed into HAp by a hydrothermal reaction.

### Experimental Example 1. Cytotoxicity measurement

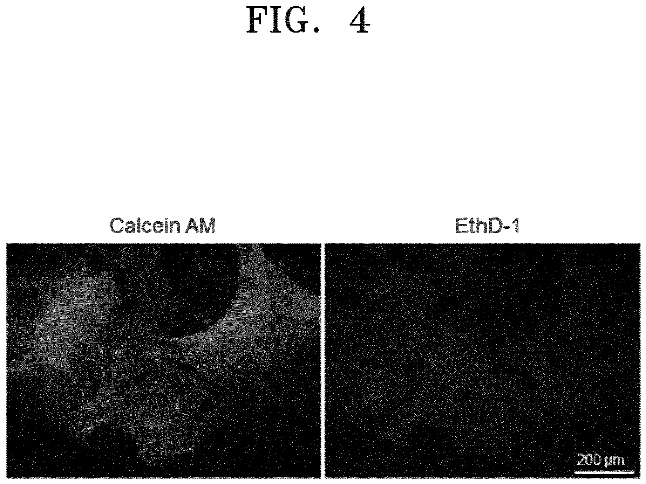

To evaluate cytotoxicity of the foraminifera-derived HAp particles, the live/dead staining analysis was performed as described in Reference Examples, and results are shown in FIG. 4.

FIG. 4 shows the images of the cytotoxicity results measured from hydroxyapatite according to an embodiment.

As shown in FIG. 4, the survived hMSCs were stained green so that most of the cells present on the particles were stained green, whereas dead cells that were stained red were not observed. These results indicate that the foraminifera-derived HAp particles did not exhibit cytotoxicity.

### Experimental Example 2. Evaluation of cell proliferation

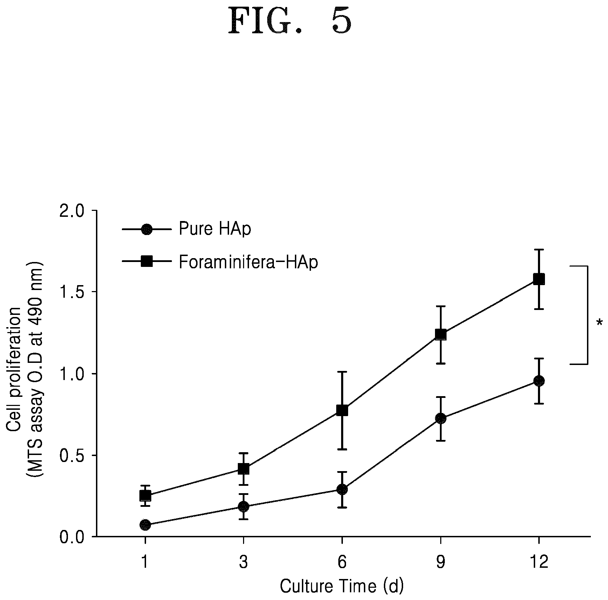

As described in Reference Examples, the cell proliferation of hMSCs in foraminifera-derived HAp was measured by using MTS mitochondrial activity-based assay. Results are shown in FIG. 5.

FIG. 5 is a graph showing the cell proliferation results measured from hydroxyapatite according to an embodiment.

As shown in FIG. 5, the hMSCs grew well over time in both pure HAp and foraminifera-derived HAp. In particular, after 1 day of the cell culture, the hMSCs cultured in foraminifera-derived HAp had an optical density value (0.25 ±0.10) that was significantly higher than an optical density value (0.07 ± 0.01) of the hMSCs cultured in pure HAp. These results indicate that foraminifera-derived HAp may be more advantageous for initial cell adhesion than pure HAp. In addition, the optical density of the hMSCs with respect to the foraminifera-derived HAp particles was significantly higher than the optical density of pure HAp at all experimental time points.

### Experimental Example 3. Observation of cell adhesion and cell infiltration

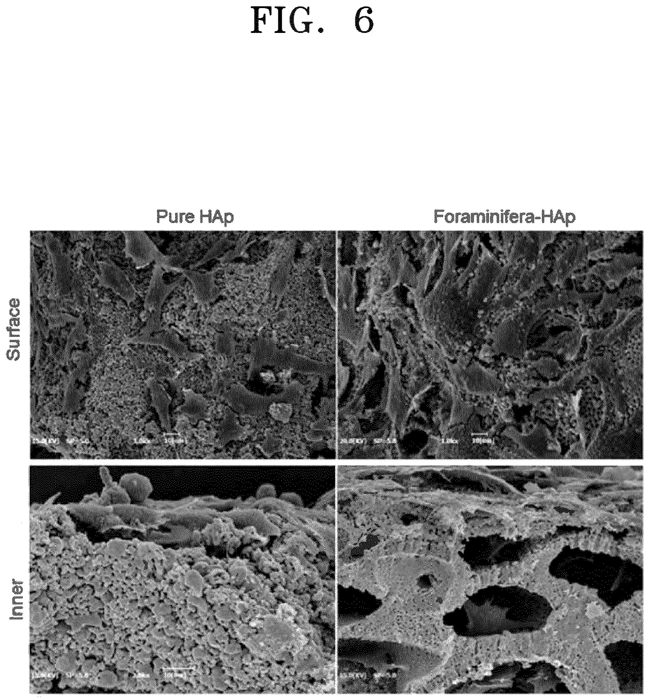

By using the methods described in Reference Examples, the cell adhesion and infiltration of the hMSCs with respect to the pure HAp particles and the foraminifera-derived HAp particles were observed by using SEM on the 5^{th} day of the cell culture, and results are shown in FIG. 6.

FIG. 6 is the SEM image showing the cell adhesion and infiltration results of hydroxyapatite according to an embodiment.

As shown in FIG. 6, the hMSCs were attached infrequently to the surface of the pure HAp particles only, and grew thereon, whereas a number of the hMSCs were attached to the surface of the foraminifera-derived HAp particles, compared to the hMSCs attached to the surface of the pure HAp particles. The infiltrated and attached hMSCs were also observed inside the chambers of the foraminifera-derived HAp particles. These results indicate that the particle structure of hydroxyapatite according to an embodiment provides a favorable environment for the cell adhesion.

### Experimental Example 4. Analysis of osteoblast differentiation ability

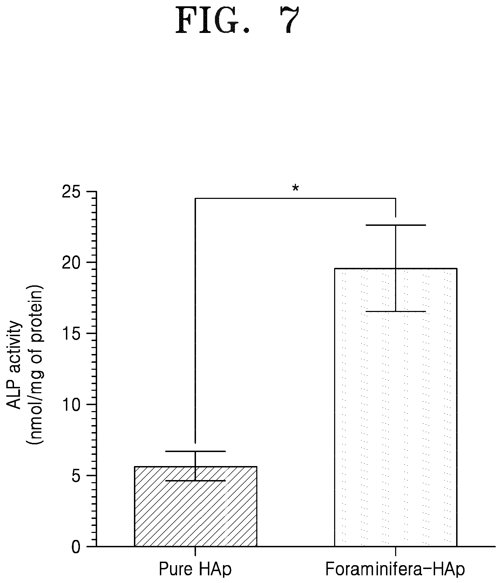

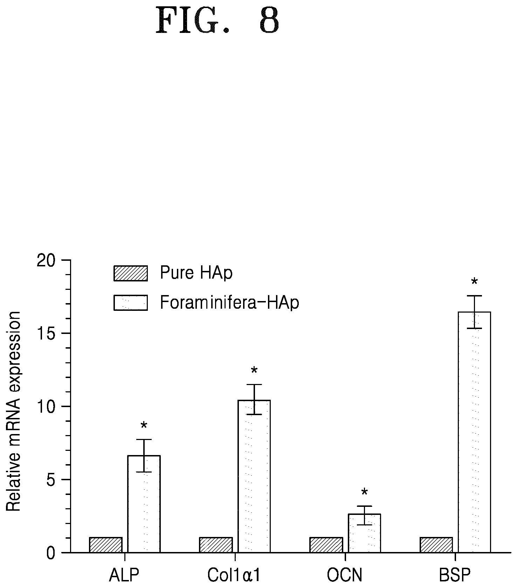

To analyze the osteoblast differentiation ability, the ALP activity and qRT-PCR were performed as described in Reference Examples to detect osteoblast marker genes, such as ALP, Colla1, OCN, and BSP. Results are shown in FIGS. 7 and 8.

FIG. 7 is a graph showing the ALP activity analysis results of hydroxyapatite according to an embodiment.

FIG. 8 is a graph showing the expression levels of osteoblast marker genes in hydroxyapatite according to an embodiment.

As shown in FIG. 7, when the cells were cultured in the medium supplemented with the osteogenic stimulator, the ALP activity was measured with 19.59 ± 3.06 nmol/mg of proteins in the foraminifera-derived HAp particles. This measurement was found to be approximately 3.4 times higher than the ALP activity of the cells cultured in pure HAp (measured with 5.69 ± 0.68 nmol/mg of proteins).

Also, as shown in FIG. 8, the mRNA expression levels of ALP, Colla1, OCN, and BSP in the foraminifera-derived HAp particles were about 6.6 times, about 10.5 times, about 2.6 times, and about 16.5 times higher, respectively, than the mRNA expression levels thereof in the pure HAp particles.

These results suggest that hydroxyapatite according to an embodiment had significant osteoblast differentiation ability compared to pure hydroxyapatite.

### Experimental Example 5. Analysis of in vivo osteoanagenesis ability

To evaluate in vivo osteoanagenesis ability of foraminifera-derived HAp prepared according to Examples above, the Micro-CT evaluation and histological evaluation were performed.

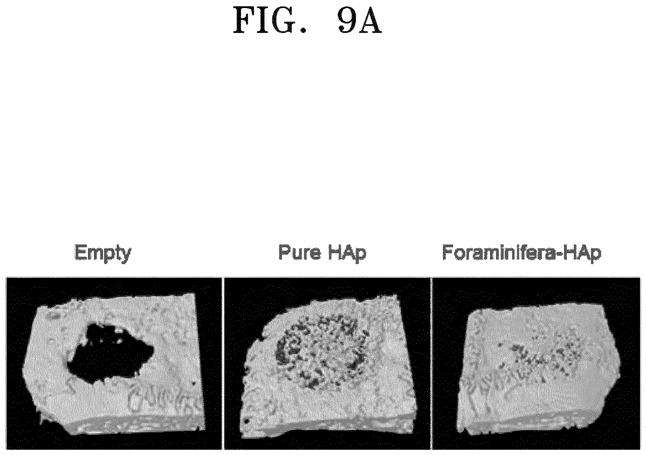

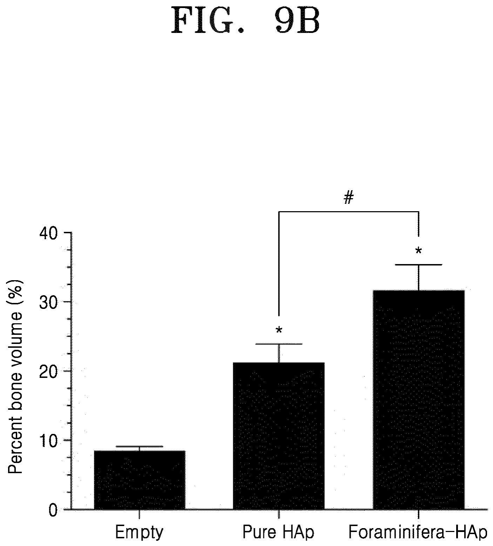

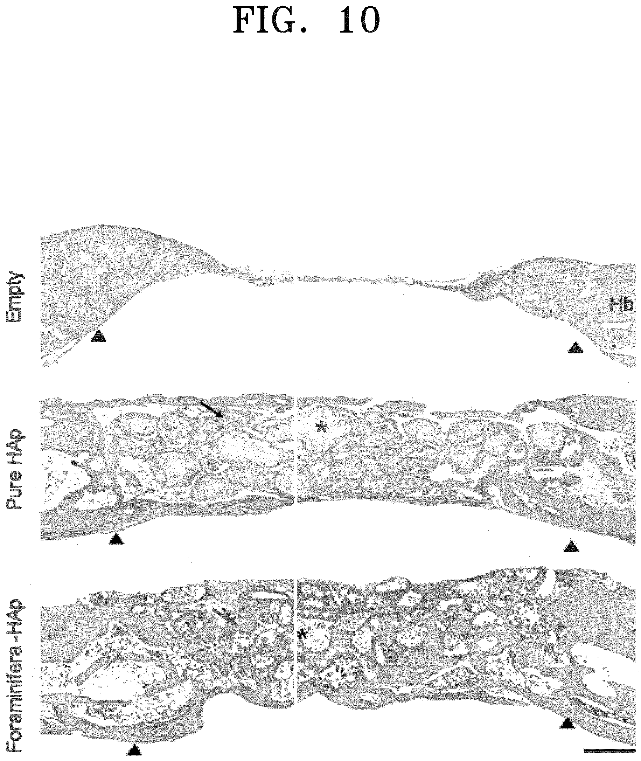

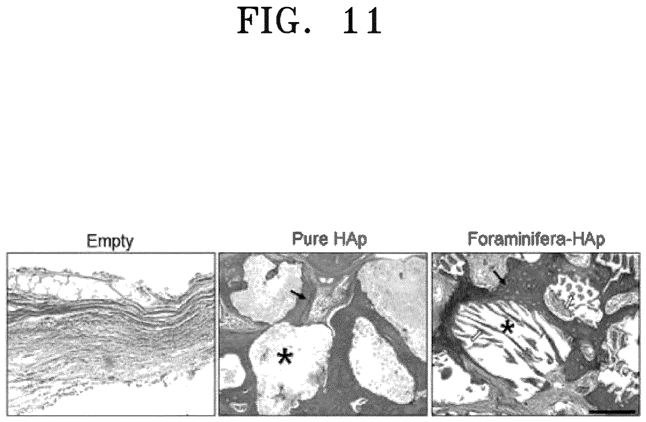

In detail, as described in Reference Examples, 3D micro-CT images were obtained at the 8^{th} week after the graft, and results are shown in FIG. 9. On the 8^{th} week after the graft, the bone defect site was stained according to the H&E staining and Goldner's Masson trichrome staining methods, and results are respectively shown in FIGS. 10 and 11.

FIG. 9 shows a figure (A) showing the micro-CT results at the 8^{th} week of in vivo graft of hydroxyapatite according to an embodiment and a graph (B) showing quantified data thereof.

FIG. 10 shows the H&E staining results showing in vivo osteoanagenesis effects of hydroxyapatite according to an embodiment.

FIG. 11 shows the Goldner's Masson trichrome staining results showing in vivo osteoanagenesis effects of hydroxyapatite according to an embodiment.

As shown in FIG. 9A, in a control group (also referred to as an empty group which does not undergo graft), new bones were regenerated at the edge of the defect site. However, in groups where pure HAp and foraminifera-derived HAp were grafted, the generation of new bones was observed not only at the edge of the defect site but also inside the grafted HAp particles. In particular, the newly formed bones in the foraminifera-derived HAp-grafted group covered the surface of the defect site. In addition, as shown in FIG. 9B, the percent bone volume of foraminifera-derived HAp (31.54 ± 3.61 %) was significantly higher than that of the control group (or empty group) (8.43 ± 0.52 %) and pure HAp (21.18 ± 2.61 %).

Also, as shown in FIG. 10, it was observed that, in all the control group (also referred to as an empty group which does not undergo graft), the pure HAp-grafted group, and the foraminifera-derived HAp-grafted group, new bones were formed at the edge of the defect site. Here, in all the grafted groups, the newly formed bones and the defects tended to be merged. In particular, it was observed that, in the pure HAp-grafted group and the foraminifera-derived HAp-grafted group, new bones were formed at the edge of the defect site, and that the new bones were formed over the outer surface of the HAp particles, resulting in bone marrow cavity-like morphology. It was also found that the mature new bones were formed at a substantial thickness in the foraminifera-derived HAp-grafted group.

As shown in FIG. 11, it was observed that, in the pure HAp-grafted group and the foraminifera-derived HAp-grafted group, new formed bones were formed not only in the peripheral region, but also in the central region of the defect site. Meanwhile, in the control group (also referred to as an empty group which does not undergo graft), only loose connective tissues were observed in the central region of the defect site. When compared with other groups, the foraminifera-derived HAp-grafted group showed a significantly large amount of mature newly formed bones. Also, only in the foraminifera-derived HAp-grafted group, it was found that new bones were formed in a similar manner with ingrowth tissues inside the porous chamber. These results indicate that the foraminifera-derived HAp particles not only stimulated the formation of new bones, but also supported the infiltration of newly formed bones inside the chamber structures.

## Claims

1. A bone graft material comprising hydroxyapatite, wherein the hydroxyapatite comprises a plurality of chambers separated by partition walls, and the partition walls comprise a plurality of pores,
wherein the hydroxyapatite has 40,000 to 60,000 uniform chambers per 1 cm² of the hydroxyapatite surface, and
wherein the hydroxyapatite is prepared by the method comprising:
pretreating foraminifera;
preparing hydroxyapatite by performing a hydrothermal reaction on the pretreated exoskeleton of foraminifera at 100 °C to 300 °C for 12 hours to 36 hours; and
sintering the hydroxyapatite,
wherein the sintering of the hydroxyapatite is to perform the following process one or more times:
raising the temperature of the hydroxyapatite to 400-800 °C at a rate of 1-20 °C/min, and then maintaining the temperature for 1-8 hours; and then
raising the temperature of the hydroxyapatite to 600-1500 °C at a rate of 1-20 °C/min, and then maintaining the temperature for 1-8 hours; and then
cooling to room temperature.

2. The bone graft material of claim 1 wherein the foraminifera is Baculogypsina sphaerulata, Baculogypsina bonarellii, Baculogypsina gallowayi, Baculogypsina lenticulate, Baculogypsina meneghinii, Baculogypsina saoneki, or Baculogypsina sphaerica.

3. The bone graft material of claim 1, wherein the bone graft material comprises a plurality of the hydroxyapatite particles, and the size of the particles is 100 µm to 4,000 µm.

4. The bone graft material of claim 1, wherein the chamber has a diameter in a range of 10 µm to 80 µm, the partition wall has a thickness in a range of 5 µm to 15 µm, or the pore has a diameter in a range of 0.1 µm to 3 µm.

5. The bone graft material of claim 1, wherein the particle of the hydroxyapatite comprises 0.5 weight% to 10 weight% of magnesium ions, 0.2 weight% to 10 weight% of silicon ions, or 0.1 weight% to 5 weight% of strontium ions.

6. The bone graft material of claim 1, wherein the bone graft material is for treating a bone defect.

7. A method of preparing the bone graft material of claim 1, the method comprising:
pretreating foraminifera; and
preparing hydroxyapatite by performing a hydrothermal reaction on the pretreated exoskeleton of foraminifera at 100 °C to 300 °C for 12 hours to 36 hours; and
sintering the hydroxyapatite,
wherein the sintering of the hydroxyapatite is to perform the following process one or more times:
raising the temperature of the hydroxyapatite to 400-800 °C at a rate of 1-20 °C/min, and then maintaining the temperature for 1-8 hours; and then
raising the temperature of the hydroxyapatite to 600-1500 °C at a rate of 1-20 °C/min, and then maintaining the temperature for 1-8 hours; and then
cooling to room temperature.

## Patentansprüche

1. Knochentransplantatmaterial umfassend Hydroxylapatit, wobei das Hydroxylapatit eine Vielzahl von Kammern umfasst, die durch Trennwände voneinander getrennt sind, und wobei die Trennwände eine Vielzahl von Poren umfassen,
wobei das Hydroxylapatit 40.000 bis 60.000 gleichförmige Kammern pro 1 cm² der Oberfläche des Hydroxylapatits aufweist, und
wobei das Hydroxylapatit durch ein Verfahren hergestellt wird, das umfasst:
Vorbehandeln Foraminiferen;
Herstellen Hydroxylapatit durch Durchführen einer hydrothermalen Reaktion an dem vorbehandelten Exoskelett von Foraminiferen bei 100 °C bis 300 °C für 12 Stunden bis 36 Stunden; und
Sintern des Hydroxylapatits,
wobei das Sintern des Hydroxylapatits den folgenden Prozess einmal oder mehrmals durchführen muss:
Erhöhen der Temperatur des Hydroxylapatits auf 400-800 °C mit einer Geschwindigkeit von 1-20 °C/min, und dann Halten der Temperatur für 1-8 Stunden; und dann
Erhöhen der Temperatur des Hydroxylapatits auf 600-1500 °C mit einer Geschwindigkeit von 1-20 °C/min, und dann Halten der Temperatur für 1-8 Stunden; und dann
Abkühlen auf Raumtemperatur.

2. Knochentransplantatmaterial nach Anspruch 1, wobei die Foraminiferen Baculogypsina sphaerulata, Baculogypsina bonarellii, Baculogypsina gallowayi, Baculogypsina lenticulate, Baculogypsina meneghinii, Baculogypsina saoneki oder Baculogypsina sphaerica sind.

3. Knochentransplantatmaterial nach Anspruch 1, wobei das Knochentransplantatmaterial eine Vielzahl von Hydroxylapatitpartikeln umfasst und die Partikelgröße zwischen 100 µm und 4.000 µm liegt.

4. Knochentransplantatmaterial nach Anspruch 1, wobei die Kammer einen Durchmesser im Bereich von 10 µm bis 80 µm aufweist, die Trennwand eine Dicke im Bereich von 5 µm bis 15 µm aufweist oder die Pore einen Durchmesser im Bereich von 0,1 µm bis 3 µm aufweist.

5. Knochentransplantatmaterial nach Anspruch 1, wobei das Partikel aus Hydroxylapatit 0,5 Gew.-% bis 10 Gew.-% Magnesiumionen, 0,2 Gew.-% bis 10 Gew.-% Siliziumionen oder 0,1 Gew.-% bis 5 Gew.-% Strontiumionen umfasst.

6. Knochentransplantatmaterial nach Anspruch 1, wobei das Knochentransplantatmaterial zur Behandlung eines Knochendefekts verwendet wird.

7. Verfahren zur Herstellung des Knochentransplantatmaterials nach Anspruch 1, umfassend:
Vorbehandeln Foraminiferen; und
Herstellen Hydroxylapatit durch Durchführen einer hydrothermalen Reaktion an dem vorbehandelten Exoskelett der Foraminiferen bei 100 °C bis 300 °C für 12 Stunden bis 36 Stunden; und
Sintern des Hydroxylapatits,
wobei das Sintern des Hydroxylapatits den folgenden Prozess einmal oder mehrmals durchführen muss:
Erhöhen der Temperatur des Hydroxylapatits auf 400-800 °C mit einer Geschwindigkeit von 1-20 °C/min, und dann Halten der Temperatur für 1-8 Stunden;
Erhöhen der Temperatur des Hydroxylapatits auf 600-1500 °C mit einer Geschwindigkeit von 1-20 °C/min, und dann Halten der Temperatur für 1-8 Stunden; und dann
Abkühlen auf Raumtemperatur.

## Revendications

1. Matière de greffe osseuse comprenant de l'hydroxyapatite, dans laquelle l'hydroxyapatite comprend une pluralité de chambres séparées par des parois de séparation, et les parois de séparation comprennent une pluralité de pores,
dans laquelle l'hydroxyapatite présente de 40 000 à 60 000 chambres uniformes par 1 cm de surface d'hydroxyapatite, et
dans laquelle l'hydroxyapatite est préparée par le procédé comprenant : un prétraitement de foraminifères ;
la préparation d'hydroxyapatite par réaction hydrothermale sur l'exosquelette prétraité de foraminifères à une température de 100 °C à 300 °C pendant 12 heures à 36 heures ; et
le frittage de l'hydroxyapatite,
dans lequel le frittage de l'hydroxyapatite consiste à effectuer une ou plusieurs fois le procédé suivant :
porter la température de l'hydroxyapatite à 400-800 °C à une vitesse de 1-20 °C/min, et puis maintenir cette température pendant 1-8 heures ; et puis
porter la température de l'hydroxyapatite à 600-1500 °C à une vitesse de 1-20 °C/min, puis maintenir cette température pendant 1-8 heures ; et ensuite
refroidir à température ambiante.

2. La matière de greffe osseuse selon la revendication 1, dans laquelle les foraminifères sont Baculogypsina sphaerulata, Baculogypsina bonarellii, Baculogypsina gallowayi, Baculogypsina lenticulate, Baculogypsina meneghinii, Baculogypsina saoneki ou Baculogypsina sphaerica.

3. La matière de greffe osseuse selon la revendication 1, dans laquelle la matière de greffe osseuse comprend une pluralité de particules d'hydroxyapatite, et la taille des particules est comprise entre 100 µm et 4 000 µm.

4. La matière de greffe osseuse selon la revendication 1, dans laquelle la chambre présente un diamètre compris entre 10 µm et 80 µm, la paroi de séparation présente une épaisseur comprise entre 5 µm et 15 µm, ou le pore présente un diamètre compris entre 0,1 µm et 3 µm.

5. La matière de greffe osseuse selon la revendication 1, dans laquelle la particule d'hydroxyapatite comprend de 0,5 % en poids à 10 % en poids d'ions magnésium, de 0,2 % en poids à 10 % en poids d'ions silicium, ou de 0,1 % en poids à 5 % en poids d'ions strontium.

6. La matière de greffe osseuse selon la revendication 1, dans laquelle la matière de greffe osseuse est destinée au traitement d'un défaut osseux.

7. Un procédé de préparation de la matière de greffe osseuse selon la revendication 1, ledit procédé comprenant :
le prétraitement de foraminifères ; et
la préparation d'hydroxyapatite par réaction hydrothermale sur l'exosquelette prétraité de foraminifères à une température de 100 °C à 300 °C pendant 12 heures à 36 heures ;
et le frittage de l'hydroxyapatite,
dans lequel le frittage de l'hydroxyapatite consiste à effectuer une ou plusieurs fois le procédé suivant :
porter la température de l'hydroxyapatite à 400-800 °C à une vitesse de 1-20 °C/min, et puis maintenir cette température pendant 1-8 heures ; et puis
porter la température de l'hydroxyapatite à 600-1500 °C à une vitesse de 1-20 °C/min, et puis maintenir cette température pendant 1-8 heures ;et ensuite
refroidir à température ambiante.
